# EUROPEAN PATENT SPECIFICATION

(11) **EP 4 083 218 B1**
(45) Date of publication and mention of the grant of the patent: **24.09.2025**
(21) Application number: 22167881.6
(22) Date of filing: 12.04.2022
(51) Int. Cl.: C12N 15/87, C12Q 1/68, C12M 1/42, G01N 33/50, G01N 33/487, G01N 27/30, G01N 33/483

(54) **METHOD FOR LABELLING NUCLEIC ACIDS**
VERFAHREN ZUR MARKIERUNG VON NUKLEINSÄUREN
PROCÉDÉ D'ÉTIQUETAGE D'ACIDES NUCLÉIQUES

(30) Priority: 14.04.2021 EP 21168293
(43) Date of publication of application: 02.11.2022
(73) Proprietor: Imec VZW, 3001 Leuven (BE)
(72) Inventor: BRAEKEN, Dries, Leuven 3001 (BE)
(74) Representative: Winger

(56) References cited:
- US-A1- 2019 177 800
- TILAK JAIN ET AL: "Microelectrode Array (MEA) Platform for Targeted Neuronal Transfection and Recording", IEEE TRANSACTIONS ON BIOMEDICAL ENGINEERING, IEEE, USA, vol. 55, no. 2, 1 February 2008 (2008-02-01), pages 827 - 832, XP011200278, ISSN: 0018-9294, DOI: 10.1109/TBME.2007.914403
- BRUNO GIULIA ET AL: "Microfluidic Multielectrode Arrays for Spatially Localized Drug Delivery and Electrical Recordings of Primary Neuronal Cultures", FRONTIERS IN BIOENGINEERING AND BIOTECHNOLOGY, vol. 8, 1 January 2020 (2020-01-01), pages 626, XP055893718, DOI: 10.3389/fbioe.2020.00626

## Description

### Technical field of the invention

The present invention relates to the field of nucleic acid labelling (e.g., barcoding), and more particularly to the field of single-cell nucleic acid sequencing, such as transcriptome sequencing.

### Background of the invention

In precision medicine, medical decisions, treatments, practices, or products are tailored to a subgroup of patients, instead of using a one-drug-fits-all model. Cell transcriptome RNA sequencing has become a very powerful technique in that field. It is the gold standard for defining cell states and phenotypes. It allows examining the expression profiles of genes of interest by sequencing RNA molecules present in cells of the patient. Typically, cells (e.g., fibroblasts or blood cells) are taken out of the patient and are cultured on a chip. Eventually, these cells may be reprogrammed to become different types of cells, such as neurons. This is especially useful since the brain of a live patient is typically not accessible for neural tissue extraction or detailed investigation in situ. The transcriptome of these cells can then be acquired. Typically, this is done in bulk, i.e., on a large number of cells originating in a single patient but lumped together. This means that at a certain moment, the cell culture is stopped, the cells are harvested and mixed, their RNA is taken out and sequenced. As a result, the transcriptome obtained is statistical data representing a large number of cells. Single-cell isolation remains difficult and needs cell detachment from the neighboring cells in the cell culture. Although this technique allows you to group certain cells by phenotype, it is not possible to trace back which phenotype was expressed by which cell in the cell culture.

RNA sequencing methods depend on poly-A tail capture to enrich mRNA and deplete abundant and uninformative rRNA, although other types of RNA can also be captured today. Spatial transcriptomics resolves RNA sequence data in individual tissue sections or cell cultures. This is done by attaching barcoded oligo(dT) primers at various specific locations of the surface of a microscope slide to encode the positional information after sequencing. Since different positions are barcoded differently, spatial information can be retrieved after sequencing. A challenge here is to obtain a high-density of primers, and the difficulty to capture efficiently RNA from the cell culture or tissue above.

Acquiring transcriptome information at a single-cell resolution is today not possible since most transcriptome analysis is done in bulk. With high-resolution spatial transcriptomics, one could achieve single cell or even subcellular resolution, but the drawback is that you need specific slides with barcodes pre-printed on them or you need to use beads or other vehicles. Also, the method is not very versatile. In principle, one can take out the mRNA from single cells by patch pipettes (Patch sequencing), and then perform sequencing. This method is, however, not very scalable.

For all cells, but in particular, for cardiac cells and neurons, an electrophysiological read-out of the state of the cells of which the transcriptome is obtained is of great importance. This permits to link the electrophysiology of the cell to its expression profile. However, acquiring a combination of electrophysiological and transcriptome information at a single-cell resolution is today not possible.

Also, current techniques only give the transcriptome at a given time. How that transcriptome evolves in time is not accessible.

Similar issues exist with cancer biopsies where characterizing the heterogeneity of cancer cell genomes is important. For such applications, both mRNA and DNA sequencing are relevant. An additional issue in that field is that it is not currently possible to sequence DNA and mRNA at the same time while simultaneously retaining where these DNAs and mRNAs were spatially located.

Tilak Jain et al, IEEE transactions on biomedical engineering, IEEE, USA, vol. 55, no.2, 1 February 2008, pages 827-832 discloses a method for labelling nucleic acids in neurons of a neuron culture. The method involves contacting the neuron culture with Alexa-Fluor-488 conjugated siRNA and applying an electrical field to the neuron, thereby increasing the permeability of the neuron membrane and allowing the Alexa-Fluor-488 conjugated siRNA to be introduced into the neuron. This does not permit a temporal analysis of the cell culture.

Bruno Giulia et al., Frontiers in bioengineering and biotechnology, vol. 8, 1 January 2020, page 626 discloses a high density microfluidic MEA capable of electroporation and performing in vitro electrophysiology in neural cell culture. This system is capable of delivery of nucleic acids and other molecules to examine gene expression on chip. It does not permit a temporal analysis of the cell culture.

US 2019/177800 discloses a method for analyzing cellular occupancy of partitions by using nucleic acid barcode sequences to generate a plurality of labeled cells. The barcodes can be introduced into the cell by electroporation and analyzed by sequencing. It does not permit a temporal analysis of the cell culture.

There is therefore a need in the art for new methods and systems overcoming at least partially one or more of these issues.

### Summary of the invention

It is an object of the present invention to provide good systems or methods for labelling (e.g. barcoding) nucleic acid in a cell.

The above objective is accomplished by a method and device according to the present invention.

In a first aspect, the present invention relates to a method for labelling (e.g. barcoding) nucleic acids in a cell of a cell culture or of a tissue, the method comprising:
a) contacting the cell culture or tissue with a first label (e.g., a barcoded nucleic acid-capturing probe) for labelling nucleic acid molecules, and
b) applying an electrical field to the cell, thereby increasing the permeability of the cell membrane, allowing the first label to be introduced into the cell.

In a second aspect, the present invention relates to a system for labelling (e.g., barcoding) nucleic acid in a cell of a cell culture or of a tissue, the system comprising a multielectrode array (MEA) having a density of electrodes equal to or higher than 1000 electrodes per square millimeter, each electrode (3) being addressable individually for applying an electric field,
wherein the system is configured to bring a label (e.g., a barcoded nucleic acid-capturing probe) in contact with a cell when a cell culture is present on the chip.

It is an advantage of embodiments of the present invention that single specific cells can be labelled (e.g., barcoded). This label (e.g., barcode) can record the exact position of the cell in the cell culture, provide a timestamp, or both.

It is an advantage of embodiments of the present invention that the transcriptome of single specific cells can be obtained instead of obtaining a statistical transcriptome lumping data of a plurality of cells. The exact position of that specific cell and, in some embodiments, the exact time at which the cell has been labelled (e.g., barcoded), is recorded by the labelling (e.g. barcoding).

It is an advantage of embodiments of the present invention that the transcriptome of single specific cells can be obtained without having to destroy the cell culture or tissue in which the cell is located.

It is an advantage of embodiments of the present invention that single specific cells and even portions of specific cells can be studied. For instance, their electrophysiological data and/or their transcriptome can be acquired, and in some embodiments, followed in time.

It is an advantage of embodiments of the present invention that one does not need elaborate techniques to keep track of where in a cell culture a nucleic acid of interest originates from. Indeed, that information is attached to the retrieved nucleic acid (typically mRNA, DNA, or both) itself by virtue of the labelling (e.g., barcoding).

It is an advantage of embodiments of the present invention that electrophysiological data and gene expression information can be acquired simultaneously with a single-cell resolution or even with a subcellular resolution.

It is an advantage of embodiments of the present invention that no expensive slides with pre-printed barcoded probes are needed.

It is an advantage of embodiments of the present invention that no extracellular vehicles such as beads are needed.

It is an advantage of embodiments of the present invention that it is easily scalable.

It is an advantage of embodiments of the present invention that it allows every cell of a cell culture or of a tissue to be quickly barcoded with information unambiguously identifying its position in the culture.

It is an advantage of embodiments of the present invention that it allows following gene expression in time at a single cell level following a stimulus.

It is an advantage of the system of the present invention that it is also suitable for introducing elements (e.g., transcription factors) in one or more specific stem cells that would enable their transformation in a differentiated cell such as a neuron or a cardiac cell.

It is an advantage of embodiments of the present invention that it makes precision medicine more easily achieved, more precise, and more affordable.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change, and evolution of devices in this field, the present concepts are believed to represent substantial new and novel improvements, including departures from prior practices, resulting in the provision of more efficient, stable, and reliable devices of this nature.

The above and other characteristics, features, and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 is a flowchart summarizing some embodiments of the first aspect of the present invention.
Fig. 2 is a schematic representation of a multielectrode array suitable for use in embodiments of the present invention.
Fig. 3 is a schematic representation of a multielectrode array sub-unit suitable for use in embodiments of the present invention.
Fig. 4 is a schematic representation of a method according to the first aspect of the present invention.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third, and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under, and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps, or components as referred to, but does not preclude the presence or addition of one or more other features, integers, steps or components, or groups thereof. The term "comprising" therefore covers the situation where only the stated features are present (and can therefore always be replaced by "consisting of" in order to restrict the scope to said stated features) and the situation where these features and one or more other features are present. The word "comprising" according to the invention therefore also includes as one embodiment that no further components are present. Thus, the scope of the expression "a device comprising means A and B" should not be interpreted as being limited to devices consisting only of components A and B. It means that with respect to the present invention, the only relevant components of the device are A and B.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures, or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures, and techniques have not been shown in detail in order not to obscure an understanding of this description.

The following terms are provided solely to aid in the understanding of the invention.

As used herein, and unless provided otherwise, the term "labelling" refers to the action of providing nucleic acid molecules, such as mRNA or DNA molecules, with a particular label. The label is typically a chemical moiety, or a molecule, suitable to serve as an identifier. The label can either attach to the nucleic acid molecule or can be incorporated in the nucleic acid molecule, e.g., during its synthesis. An example where the label is attached to the nucleic acid molecule is when the nucleic acid molecule is labelled with a barcoded nucleic acid-capturing probe. An example where the label is incorporated in the nucleic acid molecule is when the nucleic acid molecule is synthesized in presence of a metabolic label which will get incorporated in the nucleic acid molecule sequence.

As used herein, and unless provided otherwise, the term "barcoding" refers to the action of labelling nucleic acid molecules, such as mRNA or DNA molecules, with a particular barcoded nucleic acid-capturing probe. The barcoded nucleic acid-capturing probe is typically a molecule capable of hybridizing with the nucleic acid molecule and suitable to serve as an identifier. For barcoding mRNA, a barcoded nucleic acid-capturing probe can be a single strand polynucleotide (e.g. DNA) comprising an oligo(dT) sequence for hybridizing with the poly(A) tail of mRNA, a PCR handle sequence for enabling PCR amplification, and a barcode sequence suitable to serve as an identifier of either the location of the cell, the time of transfection, or both. For DNA barcoding, for example, labelled single-strand DNA or oligonucleotides can be used that hybridize to a specific DNA sequence.

As used herein, and unless provided otherwise, a metabolic label is a molecule that can be incorporated in a nucleic acid molecule sequence during its synthesis, typically rendering that nucleic acid molecule noncanonical. This molecule can, for instance, be a noncanonical nucleoside or nucleotide. Once incorporated in the nucleic acid molecule, the nucleic acid molecule can be identified by detecting the presence of the label within the sequence of the nucleic acid molecule. For instance, the label can be detected as a mutation of the nucleic acid molecule. One way to perform this is described in J. A. Schofield et al., Nature methods, vol.15 No3, March 2018, 221-22.5.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

In a first aspect, the present invention relates to a method for labelling (e.g., barcoding) nucleic acids in a cell of a cell culture or of a tissue.

The cell is a biological cell. It is preferably a eukaryotic cell. More preferably, it is an animal cell. Yet more preferably, it is a mammalian cell. Yet more preferably, it is a human cell. Most preferably it is a neuron. The present invention is well suited for the field of precision medicine, where human cells are involved. The present invention is particularly well suited for labelling (e.g., barcoding) and simultaneously recording the electrochemical activity of neurons.

How to form a cell culture on a substrate is well known to the person skilled in the art. One way to obtain a particular cell culture of animal or human cells only extractable from a body by surgery (such as neurons) is to first form a cell culture of body cells extractable from a body without surgery (such as fibroblasts or blood cells), then reprogramming the cell culture to become the cell culture of animal or human cells only extractable from a body by surgery (such as neurons or muscle cells). How to do so is well known to the person skilled in the art.

Tissues can be analyzed as well by the method of the present invention. For instance, the cell can be of a biopsy or of an organoid.

The nucleic acids may be DNA or RNA. If they are RNA, they are preferably mRNA. mRNA present in a cell gives information on the type of genes currently active in that cell. The full range of messenger RNA, or mRNA, molecules expressed by a cell is called its transcriptome. The present invention is particularly useful for obtaining a transcriptome of a cell or of a cell culture or of a tissue. If they are DNA, the present invention is particularly useful for determining the genome of a plurality of cells within a cell culture or tissue. This allows the characterization of the heterogeneity of the culture or tissue. This has applications in the field of cancer biopsies where characterizing the heterogeneity of cancer cell genomes is important.

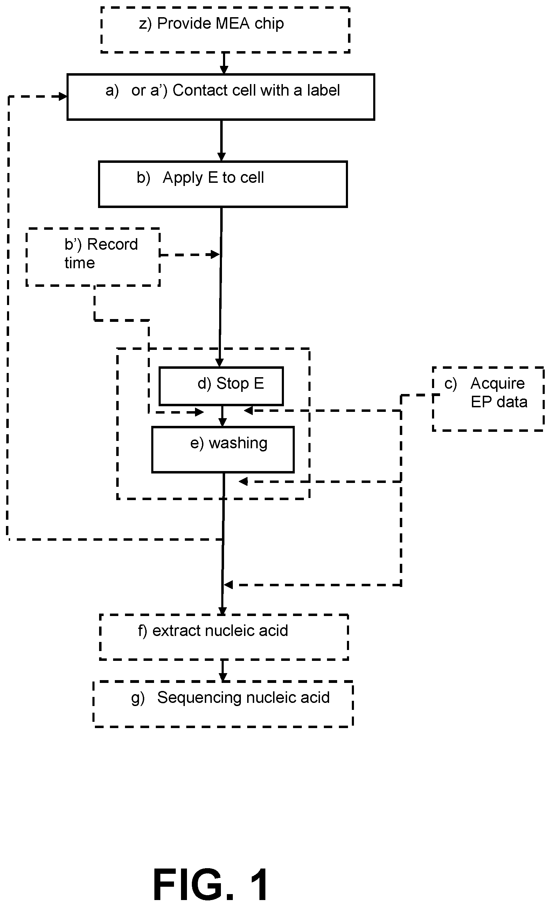

We now refer to Fig. 1. Dashed rectangles indicate optional steps. Plain-lined rectangles indicate mandatory steps.

Typically, the method of the present invention requires the presence of at least one electrode (3) configured to apply an electrical field to a cell (2) of a cell culture or of a tissue. The at least one electrode (3) is preferably also configured to measure electrical signals from the cell (2).

Preferably, a plurality of electrodes (3) is used, wherein each electrode (3) is below a different part of the cell culture or tissue. Preferably, each electrode (3) within the plurality is addressable individually to apply the electrical field. More preferably, each electrode (3) within the plurality is addressable individually to apply the electrical field and to measure electrical signals from the cell (2).

Typically, at least one electrode (3) having a cell culture thereon is provided in a step z) performed before step a) of the present invention.

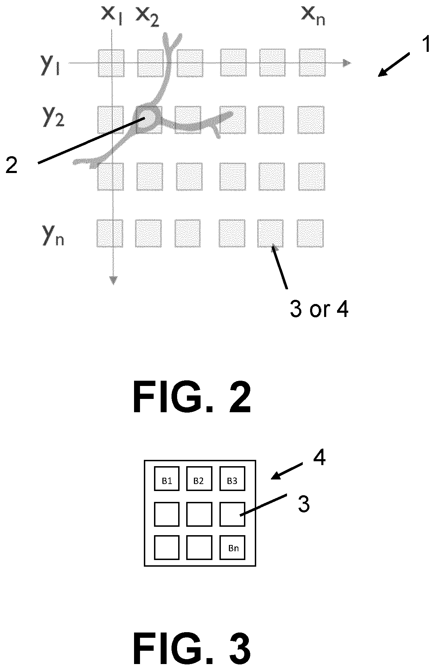

Preferably, step z) comprises providing a multielectrode array (1) having a cell culture thereon, wherein a density of electrodes (3) in the multielectrode array (1) is equal to or higher than a density of cells (2) in the cell culture or tissue, wherein applying an electrical field to the cell (2) in step b) is performed by means of an electrode (3) present under the cell (2). An example of such a multielectrode array (1) is depicted in Fig. 2. This multielectrode array (1) is depicted as having Xn columns of pixels (4) and Yn rows of pixels (4). A neuron is depicted above one of the pixels. The pixel can be one electrode (3) or a plurality of electrodes (3). Fig. 3 shows one pixel being composed of a plurality (Bn) of electrodes (3). The use of a density of electrodes (3) which is smaller or equal to the density of cells (2) is advantageous because it permits to address (and optionally record) each cell (2) individually.

In embodiments, the density of electrodes (3) may be equal to or higher than 1000 electrodes per square millimeter, preferably from 1000 to 20000 electrodes per millimeter square, preferably from 1000 to 10000 electrodes per millimeter square, or 5000 to 15000 electrodes per millimeter square.

In preferred embodiments, the multielectrode array (1) may comprise
- an active sensor area presenting a surface for cell (2) growth on the device;
- a microelectrode array (1) comprising a plurality of pixels (4) in the active sensor area, wherein each pixel comprises at least one electrode (3) at the surface, wherein the at least one electrode (3) is configured to form contact with cells (2) for providing stimulating signals to cells (2) and/or measuring electrical signals from cells (2), wherein each pixel further comprises pixel circuitry comprising at least one switch for setting a configuration of the pixel circuitry and wherein each pixel is configured to individually receive a control signal for controlling the configuration of the pixel circuitry and set a measurement modality of the pixel;
- recording circuitry having a plurality of recording channels, wherein each pixel is connected to a recording channel, the recording channel being configured to receive signals from the pixels (4) in the active sensor area, and
wherein each recording channel of the recording circuitry comprises a reconfigurable component, which is selectively controlled between being set to a first mode, in which the reconfigurable component is configured to amplify a received pixel signal, and being set to a second mode, in which the reconfigurable component is configured to selectively pass a frequency band of the received pixel signal. An example of a multielectrode array (1) usable in the method of the present invention is described in US2020018742, the content of which is incorporated herein in its entirety.

The first step of the method, i.e., step a), comprises contacting the cell culture or tissue with a first label (e.g., a barcoded nucleic acid capturing probe) (5) for labelling nucleic acids.

In embodiments, step a) may comprise providing a fluid comprising the first label (e.g. barcoded nucleic acid-capturing probe) (5) and contacting the fluid with the cell culture or tissue. The fluid is typically a liquid. This can be done in many ways. One way is to drop said fluid on the cell culture or tissue. Another way is to bring the fluid in contact with the cell culture or tissue by means of a fluidic channel fluidically connected with the cell culture. In embodiments, the fluidic channel can form part of a microfluidic system. Microfluidic systems have the advantage of permitting the delivery of the label in high concentration. It also has the advantage of permitting very good flushing and automation. A control unit can be coupled to the microfluidic system. The control unit and the microfluidic system can be configured to perform the steps of the method.

In embodiments, the fluidic channel can be transparent. This has the advantage of permitting observation of its content.

Step b) of the method comprises applying an electrical field to the cell (2), thereby increasing the permeability of the cell (2) membrane, allowing the first label (e.g. barcoded nucleic acid-capturing probe) (5) to be introduced into the cell (2). Step b) is typically performed by means of an electrode (3) present under the cell (2).

Step b) allows the label (e.g., barcoded nucleic acid capturing probe) (5) to enter the cell (2) and to label (e.g. hybridize with the nucleic acid present in the cell (2) or being incorporated in the sequence of a growing nucleic acid). Typically, step b) is performed for a time sufficient to allow labelling (e.g. hybridization) of the nucleic acid present in the cell (2) with the probe (5). Once this time has passed, the electrical field is typically interrupted.

In preferred embodiments, the method may comprise a step c) of acquiring electrophysiological data about the cell (2), wherein the method is for labelling (e.g., barcoding) nucleic acids in the cell (2) and for obtaining an electrophysiological signature of the cell (2). Typically, step c) is performed by means of the electrode (3) present under the cell (2). The same electrode (3) can, therefore, apply the electrical field and obtain an electrophysiological signature of the cell (2).

In embodiments, the method may further comprise a step d) of stopping the electrical field, a step e) of washing off any label (e.g. barcoded nucleic acid-capturing probe or noncanonical nucleoside or nucleotide) (5) present outside of the cell (2) and in contact with the cell culture or tissue, a step a') of contacting the cell culture or tissue with a further label (e.g., barcoded nucleic acid-capturing probe) (5), different from any label (e.g., barcoded nucleic acid-capturing probe) (5) previously used to contact the cell culture or tissue in previous steps of the method, and a further step b), thereby increasing the permeability of the cell (2) membrane, allowing the further label (e.g., barcoded nucleic acid- capturing probe) (5) to be introduced into the cell (2). The further label (e.g., barcoded nucleic acid-capturing probe) (5) is different from the first label (e.g. barcoded nucleic acid-capturing probe) (5) (and from any label (5) previously used to contact the cell culture or tissue in previous steps) so that it can serve as a further identifier of the cell (2) location or as an identifier of a time of transfection, or both. Typically, when the label and the further label are barcoded nucleic acid-capturing probes (5), they differ at the level of their barcode sequences. When the label and the further label are metabolic labels, the labels may differ in their chemical structures.

For instance, the first label (e.g., barcoded nucleic acid-capturing probe) (5) can serve as an indication of the location of the cell (2), such as above which row and which column of the multielectrode array (1) the cell (2) is, and the second label (e.g., a barcoded nucleic acid-capturing probe) (5) can serve as an indication of the time at which the second label (e.g., barcoded nucleic acid capturing probe) has been transfected in the cell (2). The knowledge of this time can be advantageous, for instance, if an electrophysiological measurement was performed at that time. It is also useful to know at what time was the cell (2) having a particular gene expression profile, as determined by its transcriptome.

As another example, the first label (e.g., barcoded nucleic acid-capturing probe) (5) can serve as a first indication of the location of the cell (2), such as above which column or row of the multielectrode array (1) the cell (2) is, and the second label (e.g., barcoded nucleic acid-capturing probe) (5) can serve as a second indication of the location of the cell (2), such as, respectively, above which row or column of the multielectrode array (1) the cell (2) is. Using a same first label (e.g., barcoded nucleic acid-capturing probe) (5) for each cell (2) above a same column of a multielectrode array (1), and using a same second label (e.g., barcoded nucleic acid-capturing probe) (5), different from the first label (barcoded nucleic acid-capturing probe) (5), for each cell (2) above a same row of a multielectrode array (1), enable the use of a limited number of labels (e.g., barcoded nucleic acid-capturing probes) (5) to specify the location of each cell (2) above the array (1). The alternative of using a different label (e.g., barcoded nucleic acid-capturing probe) (5) for each row-column combination is also possible but requires many more different labels (e.g., barcoded nucleic acid-capturing probes) (5).

Hence, in embodiments, when a multielectrode array (1) is used, the array (1) may comprise a plurality of columns and rows of electrodes (3), wherein step b) is performed simultaneously on a plurality of cells (2) by means of a plurality of electrodes (3) of a same column or row, thereby increasing the permeability of the cell (2) membranes of the plurality of cells (2), thereby allowing a same label (e.g., barcoded nucleic acid-capturing probe) (5) to be introduced into the plurality of cells (2). This has the advantage of limiting the number of times step b) has to be performed. This also has the advantage of limiting the number of different labels (barcoded nucleic acid-capturing probes) (5) that have the be used. In such embodiments, step b) may be repeated for each column and each row of the array (1), wherein electrodes (3) of a same column or row is introduced with a same label (e.g., barcoded nucleic acid-capturing probe) (5), wherein electrodes (3) of different columns or rows are introduced with different labels (e.g., barcoded nucleic acid-capturing probes) (5).

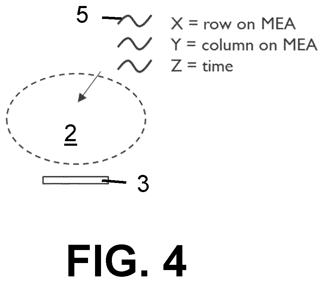

In embodiments, steps d), e), a'), and b) can be performed one or more additional times. This means that three or more labels (e.g., barcoded nucleic acid-capturing probes) (5) are used to tag a same cell (2). This typically enables to specify the location of the label (e.g. probe) (5) and at least one transfection time. This is depicted in Fig. 4. Typically, once three different labels (e.g., barcoded nucleic acid-capturing probes) (5) have been used, any further label (barcoded nucleic acid-capturing probe) (5) used will serve as a timestamp. This allows seeing how the electrophysiological state of the cell (2) and/or its transcriptome evolves over time.

In embodiments, instead of transfecting a same cell (2) repeatedly with different labels (5), it can be useful to transfect different but neighboring cells (2) with consecutive different labels (5). Although this method loses in spatial resolution, it may sometimes allow the use of more different labels (5), thereby allowing a temporal analysis of neighboring cells (2) with more time points than would be possible on a single cell (2). In such embodiments, the use of a multielectrode array (1) wherein each pixel is composed of a plurality of electrodes (3), as shown in Fig. 3, and wherein each electrode (3) is below a different cell (2), allows such a multi-time points temporal analysis. For instance, electrode (3) B1 can be used to transfect a label (5) that will serve to mark the column where the pixel is, electrode (3) B2 can be used to transfect a label (5) that will serve to mark the row where the pixel is, electrode (3) B3 can be used to transfect a label (5) that will serve as a first time stamp, and electrode (3) Bn can be used to transfect a label (5) that will serve as an n^{th-2} time stamp.

In embodiments, the method may further comprise a step b') of recording the time at which a label (e.g., a barcoded nucleic acid-capturing probe) (5) is allowed to enter into the cell (2). This permits associating a time-stamp to that label (e.g. barcode).

In embodiments, two successive different labels (e.g., barcoded nucleic acid capturing probes) (5) may be allowed to enter into the cell (2) separated by a time of at least 30 min. This permits determining the state of the cell (2) (e.g., transcriptome or electrophysiological state) at two different times separated by at least 30 min.

In embodiments, the method may further comprise f) extracting nucleic acid molecules from the cell (2), and g) sequencing the nucleic acid molecules. Typically, between step f) and step g), a step of separating the nucleic acid (e.g., mRNA) molecules from other cellular components, and a step of enriching the nucleic acid (e.g., mRNA) molecules will also be performed.

In a second aspect, the present invention relates to a system for labelling (e.g., barcoding) nucleic acids in a cell (2) of a cell culture or of a tissue, the system comprising a multielectrode array (1) having a density of electrodes (3) equal or higher than 1000 electrodes per millimeter square, each electrode (3) being addressable individually for applying an electric field,
wherein the system is configured to bring a label (e.g., a barcoded nucleic acid-capturing probe) (5) in contact with a cell (2) when a cell culture is present on the chip.

Any feature of the second aspect may be as correspondingly described for the first aspect. For instance, as indicated in the first aspect,

In preferred embodiments, the multielectrode array (1) may comprise
- an active sensor area presenting a surface for cell (2) growth on the device;
- a microelectrode array (1) comprising a plurality of pixels (4) in the active sensor area, wherein each pixel (4) comprises at least one electrode (3) at the surface, wherein the at least one electrode (3) is configured to form contact with cells (2) for providing stimulating signals to cells (2) and/or measuring electrical signals from cells (2), wherein each pixel (4) further comprises pixel (4) circuitry comprising at least one switch for setting a configuration of the pixel (4) circuitry and wherein each pixel (4) is configured to individually receive a control signal for controlling the configuration of the pixel (4) circuitry and set a measurement modality of the pixel (4);
- recording circuitry having a plurality of recording channels, wherein each pixel (4) is connected to a recording channel, the recording channel being configured to receive signals from the pixels (4) in the active sensor area, and
wherein each recording channel of the recording circuitry comprises a reconfigurable component, which is selectively controlled between being set to a first mode, in which the reconfigurable component is configured to amplify a received pixel (4) signal, and being set to a second mode, in which the reconfigurable component is configured to selectively pass a frequency band of the received pixel (4) signal. An example ofa multielectrode array (1) usable in the method of the present invention is described in US2020018742, the content of which is incorporated herein in its entirety.

### Example:

First, an MEA is provided having a culture of cells (2) thereon. This is depicted as step z) in Fig. 1. This is also schematized in Fig. 2.

Second, the cell culture is contacted with a first label (e.g., a barcoded nucleic acid-capturing probe) (5) present in a liquid solution. This is depicted as step a) in Fig. 1.

Third, an electrical pulse train is applied to a first column X1 of the array (1), thereby increasing the permeability of the cell (2) membrane into each cell (2) above the first column, allowing the first label (e.g., barcoded nucleic acid-capturing probe) (5) to be introduced into each cell (2) above the first column. This is represented by step b) in Fig. 1. A certain time (e.g., one minute) is then allowed to pass to give the first label (e.g., barcoded nucleic acid-capturing probe) (5) the time to enter the cell (2) and bind to the mRNA inside the cell (2).

Fourth, the electrical pulse train is stopped (step d in Fig. 1).

Fifth, any label (e.g., barcoded nucleic acid-capturing probe) (5) outside of the cell (2) and in contact with the cell culture is washed off. This is depicted in step e) of Fig. 1.

Sixth, the process going from the second step to the fifth step is repeated for each remaining column (X2 ... Xn) and for each row of the array (1) (Y1 ... Yn), each time using a different barcoded label (e.g., nucleic acid-capturing probe) (5).

Seventh, the process going from the second step to the sixth step is repeated one or more times, but now the time is recorded (step b' in Fig. 1) somewhere between step b) and step e) and electrophysiological data (step c in Fig. 1) is acquired somewhere between step d) and either step f) or a further step a).

Eight, the cells (2) are collected and lysed (step f in Fig. 1), the mRNA is extracted and sequenced (step g in Fig. 1)

It is to be understood that although preferred embodiments, specific constructions, and configurations, as well as materials, have been discussed herein for devices according to the present invention, various changes or modifications in form and detail may be made without departing from the scope of this invention. For example, any formulas given above are merely representative of procedures that may be used. Functionality may be added or deleted from the block diagrams and operations may be interchanged among functional blocks. Steps may be added or deleted to methods described within the scope of the present invention.

## Claims

1. An *in vitro* method for labelling nucleic acids in a cell (2) of a cell culture or of a tissue, the method comprising:
a) contacting the cell culture or tissue with a first label (5) for labelling nucleic acids,
b) applying an electrical field to said cell (2), thereby increasing the permeability of the cell (2) membrane, allowing the first label (5) to be introduced into said cell (2),
d) stopping the electrical field,
e) washing off any label (5) present outside of said cell (2) and in contact with the cell culture or tissue,
a') contacting the cell culture or tissue with a further label (5), different from any label (5) previously used to contact the cell culture in previous steps of the method, and
a further step b), thereby increasing the permeability of the cell (2) membrane, allowing the further label (5) to be introduced into said cell (2).

2. The method for labelling nucleic acids in a cell (2) of a cell culture or of a tissue according to claim 1, the method comprising:
z) before step a), providing a multielectrode array (1) having a cell culture thereon, wherein a density of electrodes (3) in the multielectrode array (1) is equal to or higher than a density of cells (2) in the cell culture or tissue, wherein applying an electrical field to the cell (2) in step b) is performed by means of an electrode (3) present under said cell (2).

3. The method according to claim 1 or claim 2, wherein said cell (2) is a neuron.

4. The method according to any one of claims 1 to 3, wherein the label (5) is either a barcoded nucleic acid capturing probe (5) or a metabolic label (5).

5. The method according to any one of claims 1 to 4, further comprising a step c) of acquiring electrophysiological data about said cell (2), wherein the method is for labelling nucleic acids in said cell (2) and for obtaining an electrophysiological signature of said cell (2).

6. The method according to claim 5 as depending on claim 2, wherein step c) is performed by means of the electrode (3) present under said cell (2).

7. The method according to claim 6, comprising performing steps d), e), a'), and b) one or more additional times.

8. The method according to any one of the preceding claims, further comprising a step b') of recording the time at which a label (5) is allowed to enter into said cell (2).

9. The method according to any one of the preceding claims, as depending on claim 2, wherein the array (1) comprises a plurality of columns and rows of electrodes (3), wherein step b) is performed simultaneously on a plurality of cells (2) by means of a plurality of electrodes (3) of a same column or row, thereby increasing the permeability of the cell (2) membranes of the plurality of cells (2), thereby allowing a same label (5) to be introduced into the plurality of cells (2).

10. The method according to claim 9, wherein step b) is repeated for each column and each row of the array (1), wherein electrodes (3) of a same column or row is introduced with a same label (5), wherein electrodes (3) of different columns or rows are introduced with different labels (5).

11. The method according to any of the preceding claims wherein step a), and if present a', comprises providing a fluid comprising the first label (5) and contacting the fluid with the cell culture.

12. The method according to any one of the preceding claims, further comprising f) extracting nucleic acid molecules from said cell (2), and g) sequencing the nucleic acid molecules.

13. The method according to claim 12 for obtaining a transcriptome of said cell (2) of a cell culture or of a tissue.

14. An *in vitro* method for labeling nucleic acids in neighboring cells (2) of a cell culture or of a tissue, the method comprising:
a) contacting the cell culture or tissue with a first label (5) for labeling nucleic acids,
b) applying an electrical field to a first cell (2), thereby increasing the permeability of the membrane of the first cell (2), allowing the first label (5) to be introduced into said first cell (2),
d) stopping the electrical field,
e) washing off any label (5) present outside of said first cell (2) and in contact with the cell culture or tissue,
a') contacting the cell culture or tissue with a further label (5), different from any label (5) previously used to contact the cell culture in previous steps of the method, and
applying an electrical field to a second cell (2), neighboring the first cell (2), thereby increasing the permeability of the membrane of the second cell (2), allowing the further label (5) to be introduced into the second cell (2).

## Patentansprüche

1. Ein In-vitro-Verfahren zum Markieren von Nukleinsäuren in einer Zelle (2) einer Zellkultur oder eines Gewebes, wobei das Verfahren umfasst:
a)- Inkontaktbringen der Zellkultur oder des Gewebes mit einer ersten Markierung (5) zum Markieren von Nukleinsäuren,
b)- Anlegen eines elektrischen Feldes an die Zelle (2), wodurch die Permeabilität der Membran der Zelle (2) erhöht wird, was es ermöglicht, dass die erste Markierung (5) in die Zelle (2) eingebracht werden kann,
d)- Aufheben des elektrischen Feldes,
e)- Abwaschen jeglicher Markierung (5), die außerhalb der Zelle (2) vorhanden ist und sich mit der Zellkultur oder dem Gewebe in Kontakt befindet,
a')- Inkontaktbringen der Zellkultur oder des Gewebes mit einer weiteren Markierung (5), die sich von jeder Markierung (5) unterscheidet, die zuvor verwendet wurde, um die Zellkultur in vorherigen Schritten des Verfahrens in Kontakt zu bringen, und
einen weiteren Schritt b), wodurch die Permeabilität der Membran der Zelle (2) erhöht wird, was es ermöglicht, dass die weitere Markierung (5) in die Zelle (2) eingebracht werden kann.

2. Das Verfahren zum Markieren von Nukleinsäuren in einer Zelle (2) einer Zellkultur oder eines Gewebes nach Anspruch 1, wobei das Verfahren umfasst:
z)- vor Schritt a), Bereitstellen einer Multielektrodenanordnung (1), die auf sich eine Zellkultur aufweist, wobei eine Dichte von Elektroden (3) in der Multielektrodenanordnung (1) gleich oder höher ist als eine Dichte von Zellen (2) in der Zellkultur oder dem Gewebe, wobei das Anlegen eines elektrischen Feldes an die Zelle (2) in Schritt b) mittels einer Elektrode (3) durchgeführt wird, die unter der Zelle (2) vorhanden ist.

3. Das Verfahren nach Anspruch 1 oder Anspruch 2, wobei die Zelle (2) ein Neuron ist.

4. Das Verfahren nach einem der Ansprüche 1 bis 3, wobei die Markierung (5) entweder eine barcodierte Nukleinsäure-Einfangsonde (5) oder eine metabolische Markierung (5) ist.

5. Das Verfahren nach einem der Ansprüche 1 bis 4, das weiter einen Schritt c) des Erfassens von elektrophysiologischen Daten über die Zelle (2) umfasst, wobei das Verfahren zum Markieren von Nukleinsäuren in der Zelle (2) und zum Erhalten einer elektrophysiologischen Signatur der Zelle (2) dient.

6. Das Verfahren nach Anspruch 5 in Abhängigkeit von Anspruch 2, wobei Schritt c) mittels der Elektrode (3) durchgeführt wird, die unter der Zelle (2) vorhanden ist.

7. Das Verfahren nach Anspruch 6, das das zusätzliche ein- oder mehrmalige Durchführen der Schritte d), e), a') und b) umfasst.

8. Das Verfahren nach einem der vorstehenden Ansprüche, das weiter einen Schritt b') des Aufzeichnens des Zeitpunkts, zu dem es einer Markierung (5) ermöglicht wird, in die Zelle (2) einzudringen, umfasst.

9. Das Verfahren nach einem der vorstehenden Ansprüche in Abhängigkeit von Anspruch 2, wobei die Anordnung (1) eine Vielzahl von Spalten und Zeilen von Elektroden (3) umfasst, wobei Schritt b) gleichzeitig an einer Vielzahl von Zellen (2) mittels einer Vielzahl von Elektroden (3) derselben Spalte oder Zeile durchgeführt wird, wodurch die Permeabilität der Zell- (2) Membranen der Vielzahl von Zellen (2) erhöht wird, wodurch es ermöglicht wird, dass dieselbe Markierung (5) in die Vielzahl von Zellen (2) eingebracht werden kann.

10. Das Verfahren nach Anspruch 9, wobei Schritt b) für jede Spalte und jede Zeile der Anordnung (1) wiederholt wird, wobei Elektroden (3) derselben Spalte oder Zeile mit derselben Markierung (5) eingebracht werden, wobei Elektroden (3) unterschiedlicher Spalten oder Zeilen mit unterschiedlichen Markierungen (5) eingebracht werden.

11. Das Verfahren nach einem der vorstehenden Ansprüche, wobei Schritt a) und, falls vorhanden, a' das Bereitstellen eines Fluids, das die erste Markierung (5) umfasst, und das Inkontaktbringen des Fluids mit der Zellkultur umfasst.

12. Das Verfahren nach einem der vorstehenden Ansprüche, das weiter f) das Extrahieren von Nukleinsäuremolekülen aus der Zelle (2), und g) das Sequenzieren der Nukleinsäuremoleküle umfasst.

13. Das Verfahren nach Anspruch 12 zum Erhalten eines Transkriptoms der Zelle (2) einer Zellkultur oder eines Gewebes.

14. Ein In-vitro-Verfahren zum Markieren von Nukleinsäuren in benachbarten Zellen (2) einer Zellkultur oder eines Gewebes, wobei das Verfahren umfasst:
a)- Inkontaktbringen der Zellkultur oder des Gewebes mit einer ersten Markierung (5) zum Markieren von Nukleinsäuren,
b)- Anlegen eines elektrischen Feldes an eine erste Zelle (2), wodurch die Permeabilität der Membran der ersten Zelle (2) erhöht wird, was es ermöglicht, dass die erste Markierung (5) in die erste Zelle (2) eingebracht werden kann,
d)- Aufheben des elektrischen Feldes,
e)- Abwaschen jeglicher Markierung (5), die außerhalb der ersten Zelle (2) vorhanden ist und sich mit der Zellkultur oder dem Gewebe in Kontakt befindet,
a')- Inkontaktbringen der Zellkultur oder des Gewebes mit einer weiteren Markierung (5), die sich von jeder Markierung (5) unterscheidet, die zuvor verwendet wurde, um die Zellkultur in vorherigen Schritten des Verfahrens in Kontakt zu bringen, und
Anlegen eines elektrischen Feldes an eine zweite Zelle (2), die der ersten Zelle (2) benachbart ist, wodurch die Permeabilität der Membran der zweiten Zelle (2) erhöht wird, was es ermöglicht, dass die weitere Markierung (5) in die zweite Zelle (2) eingebracht werden kann.

## Revendications

1. Un procédé in vitro pour marquer des acides nucléiques dans une cellule (2) d'une culture cellulaire ou d'un tissu, le procédé comprenant :
a) mettre en contact la culture cellulaire ou le tissu avec un premier marqueur (5) pour marquer des acides nucléiques,
b) appliquer un champ électrique à ladite cellule (2), augmentant ainsi la perméabilité de la membrane de ladite cellule (2), permettant au premier marqueur (5) d'être introduit dans ladite cellule (2),
d) arrêter le champ électrique,
e) laver tout marqueur (5) présent à l'extérieur de ladite cellule (2) et en contact avec la culture cellulaire ou le tissu,
a') mettre en contact la culture cellulaire ou le tissu avec un autre marqueur (5), différent de tout marqueur (5) précédemment utilisé pour mettre en contact la culture cellulaire dans les étapes précédentes du procédé, et
une étape supplémentaire b), augmentant ainsi la perméabilité de la membrane de la cellule (2), permettant au nouvel marqueur (5) d'être introduit dans ladite cellule (2).

2. Le procédé pour marquer des acides nucléiques dans une cellule (2) d'une culture cellulaire ou d'un tissu selon la revendication 1, le procédé comprenant :
z) avant l'étape a), fournir un réseau multiélectrodes (1) ayant une culture cellulaire dessus, dans lequel une densité d'électrodes (3) dans le réseau multiélectrodes (1) est égale ou supérieure à une densité de cellules (2) dans la culture cellulaire ou le tissu, dans lequel l'application d'un champ électrique à la cellule (2) à l'étape b) est effectuée au moyen d'une électrode (3) présente sous ladite cellule (2).

3. Le procédé selon la revendication 1 ou la revendication 2, dans lequel ladite cellule (2) est un neurone.

4. Le procédé selon l'une quelconque des revendications 1 à 3, dans lequel le marqueur (5) est soit une sonde de capture d'acide nucléique à code-barres (5), soit un marqueur métabolique (5).

5. Le procédé selon l'une quelconque des revendications 1 à 4, comprenant en outre une étape c) d'acquisition de données électrophysiologiques concernant ladite cellule (2), dans lequel le procédé est destiné à marquer des acides nucléiques dans ladite cellule (2) et à obtenir une signature électrophysiologique de ladite cellule (2).

6. Le procédé selon la revendication 5 dépendant de la revendication 2, dans lequel l'étape c) est effectuée au moyen de l'électrode (3) présente sous ladite cellule (2).

7. Le procédé selon la revendication 6, comprenant l'exécution des étapes d), e), a'), et b) une ou plusieurs fois supplémentaires.

8. Le procédé selon l'une quelconque des revendications précédentes, comprenant en outre une étape b') d'enregistrement du moment auquel un marqueur (5) est autorisé à entrer dans ladite cellule (2).

9. Le procédé selon l'une quelconque des revendications précédentes, dépendant de la revendication 2, dans lequel le réseau (1) comprend une pluralité de colonnes et de rangées d'électrodes (3), dans lequel l'étape b) est effectuée simultanément sur une pluralité de cellules (2) au moyen d'une pluralité d'électrodes (3) d'une même colonne ou rangée, augmentant ainsi la perméabilité des membranes des cellules (2) de la pluralité de cellules (2), permettant ainsi à un même marqueur (5) d'être introduit dans la pluralité de cellules (2).

10. Le procédé selon la revendication 9, dans lequel l'étape b) est répétée pour chaque colonne et chaque rangée du réseau (1), dans lequel les électrodes (3) d'une même colonne ou rangée sont introduites avec un même marqueur (5), dans lequel les électrodes (3) de différentes colonnes ou rangées sont introduites avec différents marqueurs (5).

11. Le procédé selon l'une quelconque des revendications précédentes dans lequel l'étape a), et si présente a', comprend la fourniture d'un fluide comprenant le premier marqueur (5) et la mise en contact du fluide avec la culture cellulaire.

12. Le procédé selon l'une quelconque des revendications précédentes, comprenant en outre f) l'extraction de molécules d'acide nucléique de ladite cellule (2), et g) le séquençage des molécules d'acide nucléique.

13. Le procédé selon la revendication 12 pour obtenir un transcriptome de ladite cellule (2) d'une culture cellulaire ou d'un tissu.

14. Un procédé in vitro pour marquer des acides nucléiques dans des cellules voisines (2) d'une culture cellulaire ou d'un tissu, le procédé comprenant :
a) mettre en contact la culture cellulaire ou le tissu avec un premier marqueur (5) pour marquer des acides nucléiques,
b) appliquer un champ électrique à une première cellule (2), augmentant ainsi la perméabilité de la membrane de la première cellule (2), permettant au premier marqueur (5) d'être introduit dans ladite première cellule (2),
d) arrêter le champ électrique,
e) laver tout marqueur (5) présent à l'extérieur de ladite première cellule (2) et en contact avec la culture cellulaire ou le tissu,
a') mettre en contact la culture cellulaire ou le tissu avec un autre marqueur (5), différent de tout marqueur (5) précédemment utilisé pour mettre en contact la culture cellulaire dans les étapes précédentes du procédé, et
appliquer un champ électrique à une seconde cellule (2), voisine de la première cellule (2), augmentant ainsi la perméabilité de la membrane de la seconde cellule (2), permettant au nouvel marqueur (5) d'être introduit dans la seconde cellule (2).
